# EUROPEAN PATENT APPLICATION

(11) **EP 3 789 018 A1**
(43) Date of publication of application: **10.03.2021**
(21) Application number: 19382778.9
(22) Date of filing: 09.09.2019
(51) Int. Cl.: A61K 31/05, A61K 31/075, A61P 1/16, A61P 3/06, A61P 3/10, A61P 5/06, A61P 9/10, A61P 9/12, A61P 19/02, A61P 19/10, A61P 25/16, A61P 25/28, A61P 29/00, A61P 35/00, A61K 8/34

(54) **COMPOSITION AND METHODS FOR ENHANCING OR PROMOTING THE SECRETION OF GHRELIN TO PROMOTE A HEALTHY METABOLIC AGING**

(71) Applicant: Servicio Andaluz de Salud, 41071 Sevilla (ES); Euronutra, S.L., 29590 Málaga (ES)
(72) Inventor: RODRIGUEZ DE FONSECA, Fernando, 29010 Málaga (ES); NAVARRO GALERA, Juan Antonio, 29010 Málaga (ES); BAIXERAS LLANOS, Elena, 29010 Málaga (ES); DECARA DEL OLMO, Juan Manuel, 29010 Málaga (ES); MEDINA VERA, Dina, 29010 Málaga (ES); LOPEZ-GAMBERO, Antonio Jesús, 29010 Málaga (ES); SUAREZ PEREZ, Juan, 29010 Málaga (ES); SANJUAN MERINO, Carlos, 29590 Málaga (ES)
(74) Representative: Hoffmann Eitle

(57) **Abstract**

This invention relates to the delivery of a composition, preferably a pharmaceutical composition, comprising D-pinitol, D-Chiro inositol or myo-inositol or any pharmaceutically acceptable salt thereof, for use in the treatment or prevention of disorders, diseases or conditions responsive to the stimulation of the ghrelin receptor in a subject in need thereof. In particular, for the treatment and/or prevention of disorders responsive to the positive modulation (stimulation) of the ghrelin receptor, such as diabetes, obesity-related disorders, and, most preferably, for treating or preventing age related conditions or diseases such as by promoting appetite, inhibiting insulin secretion and lowering insulin resistance, increasing growth hormone release, enhancing muscle vitality or fragility and treating or preventing sarcopenia by increasing net muscle mass, improving cognition (and/or treating or preventing diseases such as Azlheimer's disease, vascular dementia, Parkinson's Disease, and Huntington's disease) and treating or preventing age related hypertension.

## Description

### Field of the invention

This invention relates to the delivery of a composition, preferably a pharmaceutical composition, comprising D-pinitol, D-Chiro inositol or myo-inositol or any pharmaceutically acceptable salt thereof, for use in the treatment or prevention of disorders, diseases or conditions responsive to the stimulation of the ghrelin receptor in a subject in need thereof.

### Background of the invention

The aging process in humans is associated with physical decline and impairment of metabolic homeostasis (1). While physical decline has a relevant expression on increased fragility and the development of sarcopenia, a generalized loss of muscle mass and muscle cell performance, the dysregulation of the metabolic network leads to age-related increase of obesity, insulin resistance, diabetes and hypertension (2). Ageing impairs the activity of key metabolic signaling pathways and the ensuing metabolic dysregulation results in accelerated ageing. Thus the association of frailty, impaired metabolic homeostasis and hypertension increases vulnerability and limits the quality of life and indeed the life span of the elderly.

A key signaling system impaired in aging is insulin signaling. Age-related impairment in the activity of the insulin signalling pathway results in insulin resistance (1). The ensuing hyperglycemia, as a result of dysregulated glucose clearance, promotes formation of advanced glycation end products (AGEs), which in turn cause tissue damage further exacerbating metabolic dysregulation and accelerating the aging process. A key hepato-muscular loop is profoundly affected by the development of obesity and insulin resistance, where the coupling in between hepatic glucose production and glucose consumption by the muscle is dysregulated as results of the impairment on insulin signaling that leads to overproduction of hepatic glucose, while inhibits its utilization by the muscle, already affected by ageing-associated frailty (1).

Among the different metabolic signaling pathways that control energy homeostasis and muscle vitality, Ghrelin, a peptide hormone produced by specialized cells in the gastrointestinal tract, emerges as a potential target for age-associated metabolic dysregulation and frailty (3-5). Ghrelin is capable of promoting appetite, inhibiting insulin secretion, increasing growth hormone release, enhancing muscle vitality by increasing net muscle mass, and improving cognition (6-9). If we considered that aging produces loss of appetite, excessive insulin secretion an insulin resistance, muscle frailty and cognitive impairment, the physiological effects of Ghrelin is positioned to fight all of these age-associated conditions. Thus, enhancing the release of Ghrelin or the administration of Ghrelin receptor agonists haves been proposed to be used as a method for fighting the above cited age associated conditions (5), including hypertension since decreased Ghrelin levels is positively associated to age-related hypertension (10).

### Brief description of the invention

The present invention refers to a composition, preferably a pharmaceutical composition or a nutraceutical or a food composition, comprising D-pinitol, D-Chiro inositol or myo-inositol or any pharmaceutically acceptable salt thereof, for use in the treatment or prevention of disorders, diseases or conditions characterized by an impaired ghrelin secretion resulting in active ghrelin blood circulation levels, preferably plasma levels, lower than normal pre-prandial active ghrelin blood circulation, preferably plasma, reference levels in a subject in need thereof. Wherein said disorders, diseases or conditions characterized by an impaired ghrelin secretion resulting in active ghrelin blood circulation levels lower than normal pre-prandial active ghrelin blood circulation reference levels, can be determined by establishing whether such disorders, diseases or conditions are characterized by having altered concentrations of active ghrelin circulation levels as measured with standard and conventional methods such as ELISA or radio immune assays.

In the context of the present invention, "normal pre-prandial reference levels" are understood as pre-prandial reference levels in a healthy subject. Plasma Levels of Ghrelin are discussed, among others, in European Endocrinology, 2015;11(2):90-5 DOI: 10.17925/EE.2015.11.02.90
In the context of the present invention, "active ghrelin" is understood as the acylated (usually n-octanoylated) form of ghrelin. It is generated as a post-translational esterification of a fatty (n-octanoic or, to a lesser extent, n-decanoic) acid on serine residue at position 3 in the secreted Ghrelin. This acylation is necessary for the activity of ghrelin.

Preferably, said disorders, diseases or conditions characterized by an impaired ghrelin secretion resulting in active ghrelin blood circulation levels lower than normal pre-prandial active ghrelin blood circulation reference levels are age related conditions.

In the context of the present invention, "age related conditions" is understood as any physiological change or pathological disorder that is most often seen with increased senescence. Normal physiological changes associated with aging include loss of muscle mass and increased muscle frailty or mild cognitive impairment. Pathological disorders whose prevalence increased exponentially with age include: atherosclerosis, cardiovascular disease, type 2 diabetes, osteoporosis, hypertension, Alzheimer's disease, arthritis, cataracts and cancer.

Preferably, said disorders, diseases or conditions responsive to the modulation of the ghrelin receptor are age related conditions, wherein said composition is preferably for use in enhancing muscle vitality and reducing fragility by increasing net muscle mass.

Preferably, said disorders, diseases or conditions responsive to the modulation of the ghrelin receptor are age related conditions, wherein said composition is preferably for use in the treatment or prevention of sarcopenia.

In the context of the present invention, "sarcopenia" is defined as the loss of skeletal muscle mass and strength as a result of ageing.

Preferably, said disorders, diseases or conditions responsive to the modulation of the ghrelin receptor is hypertension, wherein said composition is for use in the treatment or prevention of hypertension, preferably for use in the treatment or prevention of age related hypertension.

In the context of the present invention, "age related hypertension" is understood as the increase in systolic blood pressure associated with age derived of the age associated increase in total (and renal vascular) resistance as a consequence of the progressive loss of the viscoelastic properties of conduit vessels, increased atherosclerotic arterial disease, and hypertrophy and sclerosis of muscular arteries and arterioles.

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer's disease.

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV).

Preferably, said disorders, diseases or conditions characterized by an impaired ghrelin secretion resulting in active ghrelin blood circulation levels lower than normal pre-prandial active ghrelin blood circulation reference levels are age related conditions, wherein said composition is preferably for use in the treatment or prevention of cognitive impairment or for improving cognition.

In the context of the present invention, "cognitive impairment" is defined by deficits in cognitive abilities (including learning, memory, perception, and problem solving) that are acquired (as opposed to the normal developmental aging associated "cognitive decline"), and may have an underlying brain pathology; "improving cognition" is understood as the amplification or extension of core capacities of the mind through improvement or information processing systems. Cognitive improvement can be achieved by means of interventions that include the administration of a compound included in the present patent.

Preferably, said disorders, diseases or conditions characterized by an impaired ghrelin secretion resulting in active ghrelin blood circulation levels lower than normal pre-prandial active ghrelin blood circulation reference levels are selected from the list consisting of Alzheimer's disease, vascular dementia, Parkinson's Disease, and Huntington's disease.

Preferably, said disorders, diseases or conditions characterized by an impaired ghrelin secretion resulting in active ghrelin blood circulation levels lower than normal pre-prandial active ghrelin blood circulation reference levels are selected from the list consisting of diabetes mellitus, preferably type II diabetes.

Preferably, said composition further comprises a pharmaceutically acceptable carrier.

Preferably, said composition is administered orally or via intragastric to a subject in need thereof.

In addition, the present invention further refers to:
- The non-therapeutic use of a composition comprising D-pinitol, D-Chiro inositol or myo-inositol or any acceptable salt thereof, for promoting appetite.
- The non-therapeutic use of a composition comprising D-pinitol, D-Chiro inositol or myo-inositol or any acceptable salt thereof, for increasing net muscle mass.
- The non-therapeutic use of a composition comprising D-pinitol, D-Chiro inositol or myo-inositol or any acceptable salt thereof, for improving cognition.

### Brief description of the figures

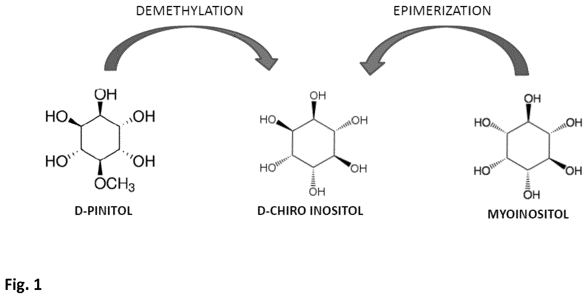

**Figure 1****.** Structure and relationship of D-Pinitol ((1S,2S,4S,5R)-6-methoxycyclohexane-1,2,3,4,5-pentol), D-Chiroinositol (1R,2R,3S,4S,5S,6S)-cyclohexane-1,2,3,4,5,6-hexol and D-myoinositol Myoinositol (1R,2S,3r,4R,5S,6s)-cyclohexane-1,2,3,4,5,6-hexol). Inositols are polialcohols with insulin-mimetic properties. D-Pinitol from natural sources (i.e. carob fruit) can be demethylated in the acid media of the stomach to be converted into D-chiro inositol). In addition, another inositol found in the diet is an isomer of D-chiro inositol that can be converted into D-chiro inositol by means of the enzymatic action of an epimerase.

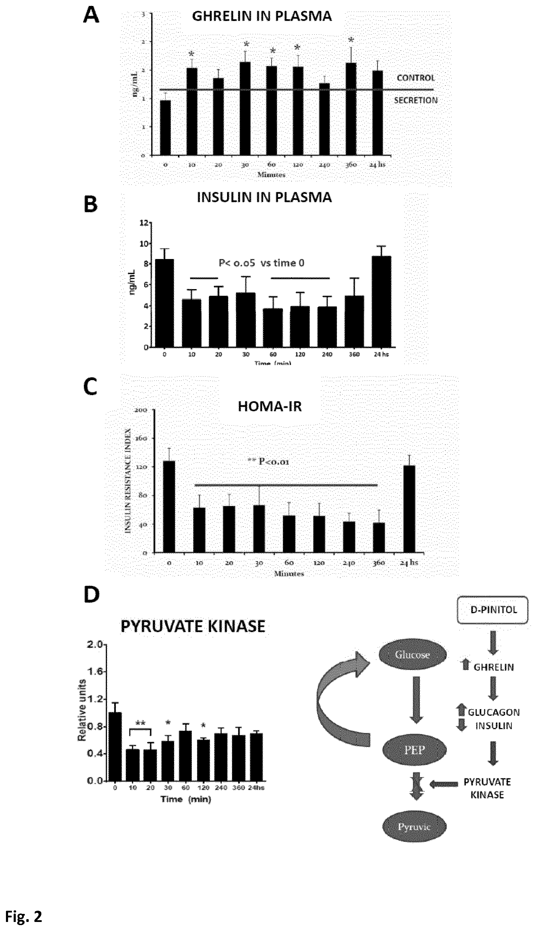

**Figure 2****.** As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

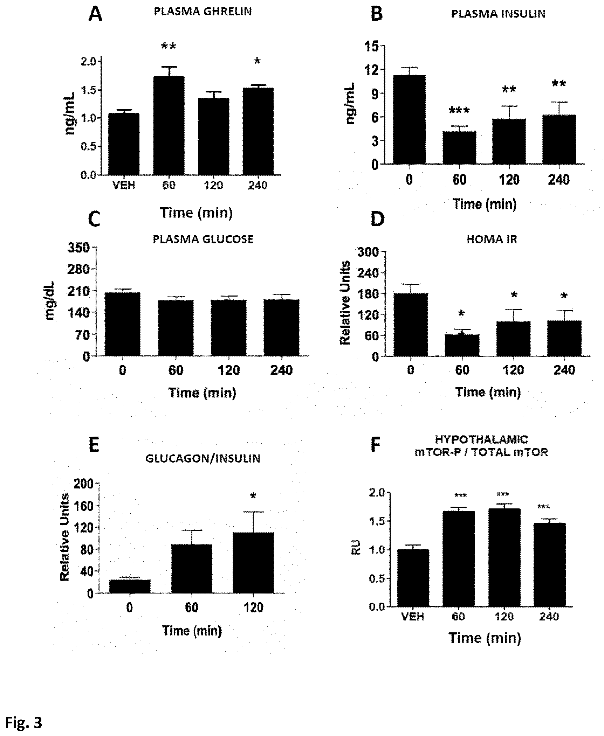

**Figure 3****.** As shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

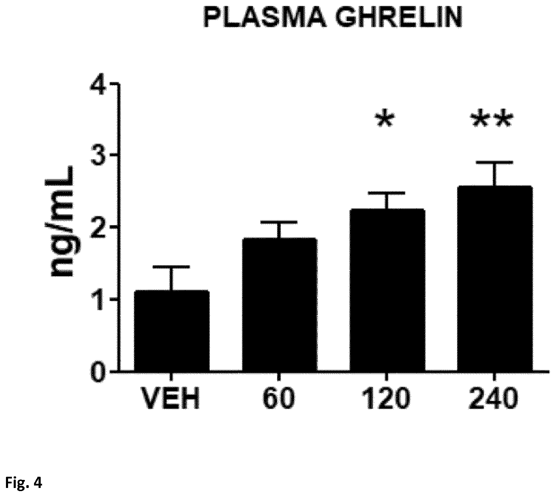

**Figure 4****.** As shown in figure 4, oral administration of D-Chiroinositol (500 mg/kg) enhances Ghrelin secretion as measured through monitorization of circulating plasma Ghrelin concentrations.

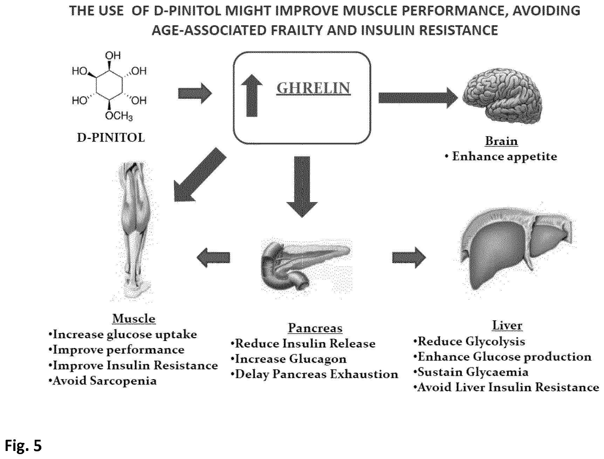

**Figure 5** illustrates a proposed model of action of inositols in metabolic aging. Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

### Description of the invention

The present invention relates to a method for increasing ghrelin levels as a result of the oral administration of D-pinitol, a natural cyclic polyol derived from plants including the pods of the carob tree, or related inositols such as D-chiro inositol or its epimer myo-inositol (figure 1). As shown in the examples included throughout the present specification, the oral administration of D-Pinitol (100 mg/kg (Figure 2) or the oral administration of 500 mg/kg of D-Chiro inositol (Figure 3), enhances ghrelin secretion and reduces circulating insulin, and further activates the phosphorylation of mTOR in the hypothalamus, a metabolic sensor needed to increase appetite (figure 3). The net effect is a reduction of the insulin demand, as reflected by the lowering of the insulin resistance index HOMA (Figures 2 & 3), but without leading to hypo- or hyperglycaemia. This is possible thanks to the direct effect of D-pinitol and D-Chiroinositol on the glucose uptake by muscle cells coupled to the net production of glucose by the liver thanks to the inhibition of the pyruvate kinase that redirects glycolisis towards glucose production in the liver (noeglucogenesis) (figure 2). Administration of D-Chiro inositol also enhances ghrelin secretion (figure 4).

In addition, and as proposed in figure 5, the use of D-Pinitol and D-Chiro inositol to promote Ghrelin secretion harmonizes insulin and glucagon secretion, reducing insulin demand, and redirecting glucose production from the liver to its utilization by the muscle. In addition, because of the known actions of ghrelin, it is expected that D-pinitol or related inositols such as D-chiro inositol or its epimer myo-inositol, increase muscle mass and vitality reducing muscle frailty. Since Ghrelin concentrations decline with age (16), these compounds will help promoting a healthy metabolic aging.

Therefore, the present invention relates to inositols such as D-pinitol, D-chiro inositol and its epimer myo-inositol (from hereinafter compounds of the present invention) for increasing ghrelin levels. Compounds of the present invention are described by the structural formulas identified in figure 1 or by any pharmaceutically acceptable salt derived therefrom.

As already indicated, the compounds of the invention are effective for increasing ghrelin levels. They are therefore useful for the treatment and/or prevention of disorders characterized by an impaired ghrelin secretion resulting in active ghrelin blood circulation levels lower than normal pre-prandial active ghrelin blood circulation reference levels, such as in age related conditions, wherein normal physiological changes associated with aging include loss of muscle mass and increased muscle frailty or mild cognitive impairment. Pathological disorders whose prevalence increased exponentially with age further include: atherosclerosis, cardiovascular disease, type 2 diabetes, osteoporosis, hypertension, Alzheimer's disease, arthritis, cataracts and cancer. Preferably, said disorders, diseases or conditions characterized by an impaired ghrelin secretion resulting in active ghrelin blood circulation levels lower than normal pre-prandial active ghrelin blood circulation reference levels is hypertension, preferably age related hypertension.

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer's disease.

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV).

Preferably, said disorders, diseases or conditions characterized by an impaired ghrelin secretion resulting in active ghrelin blood circulation levels lower than normal pre-prandial active ghrelin blood circulation reference levels are age related conditions, wherein the compounds of the invention are preferably for use in the treatment or prevention of cognitive impairment or for improving cognition.

Also preferably, said disorders, diseases or conditions characterized by an impaired ghrelin secretion resulting in active ghrelin blood circulation levels lower than normal pre-prandial active ghrelin blood circulation reference levels are selected from the list consisting of Alzheimer's disease, vascular dementia, Parkinson's Disease, and Huntington's disease.

Therefore, an aspect of the present invention provides a method for the treatment or prevention of any of the above mentioned diseases, disorders or conditions in a subject in need thereof, which comprises administering to said subject a therapeutically or prophylactically effective amount of D-pinitol, D-Chiro inositol or myo-inositol or any pharmaceutically acceptable salt thereof. The present invention also relates to methods for treating or preventing any of the above mentioned diseases, disorders or conditions in a subject in need thereof, by administering D-pinitol, D-Chiro inositol or myo-inositol or any pharmaceutically acceptable salt thereof in combination with a therapeutically or prophylactically effective amount of another agent known to be useful to treat or prevent any of said conditions or diseases. Another aspect of the present invention provides a pharmaceutical composition comprising a compound having the structural formula D-pinitol, D-Chiro inositol or myo-inositol and a pharmaceutically acceptable carrier.

Yet another aspect of the present invention relates to the use of D-pinitol, D-Chiro inositol or myo-inositol or any pharmaceutically acceptable salt thereof for the manufacture of a medicament useful for the treatment or prevention, or suppression of a disease characterized by an impaired ghrelin secretion resulting in active ghrelin blood circulation levels lower than normal pre-prandial active ghrelin blood circulation reference levels in a subject in need thereof. In particular for the treatment or prevention, or suppression of any of the above mentioned diseases, disorders or conditions.

Yet another aspect of the present invention relates to the non-therapeutic use of an amount of D-pinitol, D-Chiro inositol or myo-inositol or any salt thereof, for promoting appetite, inhibiting insulin secretion and lowering insulin resistance, increasing growth hormone release, enhancing muscle vitality or fragility by increasing net muscle mass, improving cognition and preventing age related hypertension in a subject in need thereof.

D-pinitol, D-Chiro inositol or myo-inositol or any pharmaceutically acceptable salt thereof or ester compounds can be provided in a kit. Such a kit typically contains an active compound of D-pinitol, D-Chiro inositol or myo-inositol in dosage forms for administration. A dosage form contains a sufficient amount of active compound such that a beneficial effect can be obtained when administered to a subject during regular intervals, such as 1, 2, 3, 4, 5 or 6 times a day, during the course of 1 or more days. Preferably, a kit contains instructions indicating the use of the dosage form and the amount of dosage form to be taken over a specified time period.

The term "subject" means a mammal. One embodiment of the term "mammal" is a "human," said human being either male or female. The instant compounds are also useful for treating or preventing age related conditions or diseases such as for promoting appetite, inhibiting insulin secretion and lowering insulin resistance, increasing growth hormone release, enhancing muscle vitality or fragility and treating or preventing sarcopenia by increasing net muscle mass, improving cognition and treating or preventing age related hypertension in cats and dogs. As such, the term "mammal" includes companion animals such as cats and dogs. The term "mammal in need thereof refers to a mammal who is in need of treatment or prophylaxis as determined by a researcher, veterinarian, medical doctor or other clinician.

The term "composition", as in pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier. The term "composition" is also intended to encompass nutraceutical or food compositions. A nutraceutical is a food or food product that provides health and medical benefits, including the prevention and treatment of human ailments.

It will be understood that the compounds of the present invention include hydrates, solvates, polymorphs, crystalline, hydrated crystalline and amorphous forms of the compounds of the present invention, and pharmaceutically acceptable salts thereof. The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, lithium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl- morpholine, N-ethylpiperidine, glucamine, glucosamine, histidine, hydrabamine, isopropylamine, lysine, methylglucamine, moφholine, piperazine, piperidine, polyamine resins, procaine, purines, theobromine, triethylamine, trimethylamine, tripropylamine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic acid, trifluoroacetic acid, and the like. Particularly preferred are citric, fumaric, hydrobromic, hydrochloric, maleic, phosphoric, sulfuric, and tartaric acids.

Preferably, and as already indicated throughout the present specification, it will be understood that, as used herein, references to compounds D-pinitol, D-Chiro inositol and myo-inositol are meant to also include the pharmaceutically acceptable salts, such as the hydrochloride salts. The compounds of the present invention are useful in the treatment, control or prevention of diseases, disorders or conditions characterized by an impaired ghrelin secretion resulting in active ghrelin blood circulation levels lower than normal pre-prandial active ghrelin blood circulation reference levels. In particular, the compounds of the present invention are useful in promoting a healthy metabolic aging. Such promotion is carried out by treating or preventing diseases, disorders or conditions that include, but are not limited to, pathological disorders whose prevalence increased exponentially with age such as: atherosclerosis, cardiovascular disease, type 2 diabetes, osteoporosis, hypertension, Alzheimer's disease, arthritis, cataracts and cancer. Preferably, said disorders, diseases or conditions responsive to the modulation of the ghrelin receptor is hypertension, preferably age related hypertension. Preferably, said disorders, diseases or conditions responsive to the modulation of the ghrelin receptor are age related conditions, wherein the compounds of the invention are preferably for use in inhibiting insulin secretion and lowering insulin resistance to avoid the progression of the insulin resistance syndrome, a condition where there is a progressive loss of the cellular responses to insulin that results in progressive rise of insulin levels and subsequent endocrine pancreas exhaustion. Diseases that will benefit of a reduction of excess insulin might include diabetes type 2, hypertension, dyslipidaemia, cardiovascular disease, non-alcoholic steatohepathitis and brain insulin resistance associated to Alzheimer's disease. Preferably, said disorders, diseases or conditions responsive to the modulation of the ghrelin receptor are age related conditions, wherein the compounds of the invention are for use in increasing circulating growth hormone release to compensate the physiological decline in growth hormone release associated with aging, as well as in conditions where we need to increase exercise capacity, muscle mass and bone density because of a low growth hormone secretion such as the infection with Human immunodeficiency virus (HIV). Preferably, said disorders, diseases or conditions responsive to the modulation of the ghrelin receptor are age related conditions, wherein the compounds of the invention are preferably for use in the treatment or prevention of cognitive impairment or for improving cognition. Also preferably, said disorders, diseases or conditions responsive to the modulation of the ghrelin are selected from the list consisting of Alzheimer's disease, vascular dementia, Parkinson's Disease, and Huntington's disease.

The compositions of the present invention are thus especially effective for treating Type II diabetes. The compounds or combinations of the present invention are also useful for treating and/or preventing gestational diabetes mellitus. Treatment of diabetes mellitus refers to the administration of a compound or combination of the present invention to treat diabetes. One outcome of treatment may be increasing insulin levels and increasing insulin sensitivity. Another outcome of treatment may be decreasing insulin resistance in a subject with increased insulin resistance. Prevention of diabetes mellitus refers to the administration of a compound or combination of the present invention to prevent the onset of diabetes in a subject at risk thereof.

The terms "administration of and or "administering" a compound should be understood to mean providing a compound of the invention or a prodrug of a compound of the invention to a subject in need of treatment. The administration of the compounds of the present invention in order to practice the present methods of therapy is carried out by administering a therapeutically effective amount of the compound to a subject in need of such treatment or prophylaxis. The need for a prophylactic administration according to the methods of the present invention is determined via the use of well known risk factors.

The term "therapeutically effective amount" as used herein means the amount of the active compound that will elicit the biological or medical response in a tissue, system, subject, mammal, or human that is being sought by the researcher, veterinarian, medical doctor or other clinician, which includes alleviation of the symptoms of the disorder being treated. The novel methods of treatment of this invention are for disorders known to those skilled in the art. The term "prophylactically effective amount" as used herein means the amount of the active compound that will elicit the biological or medical response in a tissue, system, subject, mammal, or human that is being sought by the researcher, veterinarian, medical doctor or other clinician, to prevent the onset of the disorder in subjects as risk for obesity or the disorder. The therapeutically or prophylactically effective amount, or dosage, of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

Administration and Dose Ranges. Any suitable route of administration may be employed for providing a subject or mammal, especially a human with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the invention are administered orally. The effective dosage of active ingredient employed may vary depending on the particular compound employed, the mode of administration, the condition being treated and the severity of the condition being treated. Such dosage may be ascertained readily by a person skilled in the art.

The magnitude of prophylactic or therapeutic dosage of the compounds of the present invention will, of course, vary depending on the particular compound employed, the mode of administration, the condition being treated and the severity of the condition being treated. It will also vary according to the age, weight and response of the individual patient. Such dosage may be ascertained readily by a person skilled in the art.

As already stated, compounds of the invention may be used in combination with other drugs that are used in the treatment/prevention/suppression or amelioration of the diseases or conditions for which compounds of the invention are useful. Such other drugs may be administered, by a route and in an amount commonly used therefor, contemporaneously or sequentially with a compound of the invention. When a compound of the invention is used contemporaneously with one or more other drugs, a pharmaceutical composition containing such other drugs in addition to the compound of the invention is preferred.

The compositions of the present invention include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy. In practical use, the compounds of the invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the typical oral dosage unit form, in which case solid pharmaceutical carriers are typically employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray. The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of the invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

Finally, compounds of the invention may also be administered as nutraceutical or food compositions such as beverages.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

The following examples serve to illustrate the present invention but do not limit the same.

### Examples

### METHODOLOGIES

### Animals and ethics statement.

Experimental procedures with animals were carried out in strict accordance with the recommendations in the European Communities directive 2010/63/EU and Spanish legislation (Real Decreto 53/2013, BOE 34/11370-11421, 2013) regulating the care and use of laboratory animals. The protocol was approved by the Ethics Committee for Animal Experiments of the University of Malaga. All studies involving animals were reported in accordance with the ARRIVE guidelines for reporting experiments involving animals (Kilkenny, C., Browne, W. J., Cuthill, I. C., Emerson, M. & Altman, D. G. Improving Bioscience Research Reporting: The ARRIVE Guidelines for Reporting Animal Research. PLoS Biol 8, e1000412, https://doi.org/10.1371/journal.pbio.1000412 (2010). All efforts were made to minimize animal suffering and to reduce the number of animals used. The experiments were performed on 4-to-5-week-old male Wistar rats (Crl:WI; Charles River Laboratories, Barcelona, Spain). The animals were under a standard 12 h light-dark cycle in a room with temperature and humidity control. All the rats were provided with water and commercially available rat standard pellet diet (STD) (3.02 kcal/g with 30 kcal% protein, 55 kcal% carbohydrates and 15 kcal% fat) ad libitum.

### Drug preparation and dose administered.

D-Pinitol (3-0-methyl-D-chiro-inositol) was generously provided by EURONUTRA SL (https://www.euronutra.com/, Málaga, Spain), in the form of crystalline fine powder (lot: PPN-M0201). For acute treatments, D-Pinitol was dissolved in water to be administered by gavage (orally) at different concentrations (100 mg/Kg and 500 mg/Kg), drug was administered at a volume of 1 ml/kg.

### Treatment guidelines.

After 18 hours of fasting, D-Pinitol dissolved in water was administered acutely by gavage (100 mg/kg in a first study, and 500 mg/kg in a subsequent study). After administration, the animals were sacrificed in groups at different times (being time 0 the administration of D-Pinitol). For the first study (100 mg/kg), groups of animals were sacrificed at times: 10, 20, 30, 60, 120, 240 and 360 minutes after administration; for the second study (500 mg/kg), animals were sacrificed at times: 60, 120 and 240 minutes after administration. As a control group, a group was administered only with water (by gavage).

### Sample collection.

Animals were anesthetized with sodium pentobarbital (50 mg/Kg, i.p.), blood, brain and liver samples were collected. Blood was centrifuged (2100 g for 8 min, 4 °C) and the plasma kept for further analysis. Liver and brain samples were flash-frozen in liquid N2, then stored at -80 °C until further analysis.

### Measurement of metabolites in plasma.

The plasma levels of insulin and ghrelin were determined with an enzyme-linked immunosorbent assay (ELISA) method using commercial kits: EMD Millipore Corporation (Billerica, MA, USA) Cat. #EZRMI-13K and Cat. #EZRGRT-91K, respectively. Glucagon levels in plasma was determined with a Glucagon Enzyme Immunoassay (EIA) Kit: Cat. #RAB0202 Sigma-Aldrich (Saint Louis, MO, USA). All serum samples were assayed in duplicate within one assay, and results were expressed in terms of the particular standard hormone.

### RNA isolation and cDNA synthesis.

Total RNA was extracted from tissue portions of liver (100-300 mg) using the Trizol® method according to the manufacturer's instructions (GIBCO BRL Life Technologies). To ensure the purity of the mRNA sequences, RNA samples were isolated with a RNeasy Minelute Cleanup Kit (Qiagen), which included digestion with DNase I column (RNase-free DNase set, Qiagen), according to the manufacturers' instructions. Total RNA was quantified using a spectrophotometer (Nanodrop 1000 Spectrophotometer, Thermo Scientific) to ensure A260/280 ratios of 1.8 to 2.0. Reverse transcription was carried out from 1 µg of RNA using the Transcriptor Reverse Transcriptase kit and random hexamer primers (Transcriptor RT, Roche Diagnostic GmbH). Negative controls included reverse transcription reactions that omitted the reverse transcriptase.

### Real-time Quantitative Polymerase Chain Reaction (qPCR) and Gene Expression Analysis.

Real-time qPCR was performed following the criteria of the MIQE guidelines (Bustin, S. A. et al. The MIQE guidelines: minimum information for publication of quantitative real-time PCR experiments. Clin Chem. 55, 611-622 (2009)). Polymerase chain reactions were carried out on the CFX96 Touch™ Real-Time PCR detection system (Bio-Rad, Hercules, CA) for each cDNA template, and amplified in 20 µl reaction volume containing 9 µl of cDNA (diluted 1/100) and 11 µl of master mix containing the primer (TaqMan, Life Technologies). The target rat gen was Pyruvate Kinase Liver/RBC (Pklr) (Supplementary Table). The primer was obtained based on TaqMan® Gene Expression Assays and the FAM™ dye label format (Life Technologies). Each reaction was run in duplicate. Cycling parameters were 50 °C for 2 min to deactivate single- and double stranded DNA containing dUTPs, 95 °C for 10 min to activate Taq DNA polymerase followed by 40 cycles at 95 °C for 15 sec for cDNA melting, and 60 °C for 1 minute to allow for annealing and the extension of the primers, during which fluorescence was acquired. Raw fluorescence data were submitted to the online Miner tool (http://www.miner.ewindup.info/) for calculation of Cq and efficiency values for each experimental set (Zhao, S. & Fernald, R. D. Comprehensive Algorithm for Quantitative Real-Time Polymerase Chain Reaction. J Comput Biol. 12, 1047-1064 (2005)). Cq values were converted into relative expression values taking into account amplification efficiencies, inter-run variations, and normalization factors by means of Biogazelle's qbasePLUS software (Biogazelle, Zwijnaarde, Belgium) using at least two reference rat genes: beta Actin (Actb) and Glyceraldehyde-3-phosphate Dehydrogenase (Gapdh) (Supplementary Table). For all reference and target gene studies, two independent biologic samples of each experimental condition were evaluated in technical duplicates. Repeatability between replicates was accepted when the ΔCq value was ≤0.7. Finally, Calibrated Normalized Relative Quantity (CNRQ) values were exported from the qbasePLUS software and investigated statistically.

**Table. Primer references for TaqMan® Gene Expression Assays (Applied Biosystems).**

| **Gene description** | **Assay ID** | **N° accession GenBank** | **Amplicon Length** |
|---|---|---|---|
| **Target genes** | | | |
| *Pklr* | Rn01455286_m1 | NC_005101.4 | 58 |

| **Reference genes** | | | |
|---|---|---|---|
| Gapdh | Rn01775763_g1 | NC_ 005103.4 | 174 |
| Actb | Rn00667869_m1 | NM_031144.2 | 91 |

### Protein extraction and Western blot analysis.

Brain extract. Frozen brain samples (17 mg per sample) were homogenized in 1mL of cold RIPA lysis buffer (50 mM Tris-HCI pH 7.4, 150 mM NaCl, 0.5% NaDOC, 1 mM EDTA, 1% Triton, 0.1% SDS, 1 mM Na3VO4, 1 mM NaF) supplemented with a protease cocktail (Roche). The suspension was incubated for 2 hours at 4 °C, followed by centrifugation at 12,000 rpm for 15 minutes at 4 °C. The supernatant was transferred to a new clean centrifuge tube, and Bradford colorimetric method was used to determine the concentration of the total protein. The protein extracts were diluted 1:1 in loading buffer (DTT 2X) and heated for 5 minutes at 99 °C before being subjected to electrophoresis.

Western Blot Analysis. The expression of protein including mTOR (289kDa) and Adaptin (100kDa) were analysed by western blot. The tissue protein (10-15µg) was subjected to electrophoresis on 4-12% Criterion XT Precast Bis-Tris gels (Bio-Rad, Hercules, CA, EE.UU.) for 30 minutes at 80V and 2 h at 150V. Proteins were transferred onto a 0.2 µm nitrocellulose membrane (Bio-Rad, Hercules, CA, EE.UU.) for 1h at 80V by wet transfer equipment. The membrane was washed twice for 5 min in TBST (10 mM Tris-HCl, 150 mM NaCl, 0.1% Tween 20, pH 7.6) and blocked with 5% BSA-TBST for one hour at room temperature on a shaker platform. Subsequently, the membrane was incubated with respective primary antibodies overnight at 4°C diluted in 2% BSA-TBST. Antibodies against p-mTOR (Ser2448) and m-TOR were purchased from Cell Signaling Technology (Danvers, Massachusetts, United States); α-Adaptin from Abcam (Cambridge, United Kingdom). The following day, the membrane was washed three times for 5 min with TBST. An appropriate HRP conjugated rabbit/mouse secondary antibody (Promega, Madison, WI, EE.UU.) was diluted 1:10000 in 2% BSA-TST and incubated with the membrane for 1h shaking at room temperature. Finally, the membrane was washed as above and exposed to chemiluminescent reagent (Santa Cruz, Biotechnology Inc., CA, EE.UU.) for 5 min. Respective membrane bound protein was then visualized by Chemiluminescence (ChemiDoc Imaging System, Bio-Rad). Bands were quantified by densitometric analysis using ImageJ software (Rasband, WS, ImageJ, US National Institutes of Health, Bethesda, MD, USA).

All data are expressed as mean ± SEM. Statistical analysis were conducted in GraphPad Prism, version 8 (GraphPad Software, Inc., La Jolla, CA). One-way analysis of variance (ANOVA) was assessed, followed by a Turkey's Multiple Comparisons Test when appropriate. P values less than 0.05 were considered significant.

### Results

### 1.1. Results of the oral administration of Pinitol (100 mg/kg) dissolved in sterile water to adult male Wistar Rats

As shown in figure 2, oral administration of Pinitol (100 mg/kg) dissolved in sterile water to adult male Wistar Rats results in a) an increase in circulating plasma Ghrelin concentration that is associated with B) decreased insulin release into the blood circulation, C) reduction of insulin resistance measured by the HOMA index, and D) Inhibition of the expression of Pyruvate Kinase, a key enzyme for diverting phosphoenolpyruvate to glucose production

Furthermore, as shown in figure 3, oral administration of Pinitol (500 mg/kg) to adult male Wistar Rats results in A) an increase in circulating plasma Ghrelin concentration that is associated with B) decreased insulin release into the blood circulation, C) maintenance of glucose levels in plasma, D) reduction of insulin resistance measured by the HOMA index, E) Increase in glucagon secretion and F) activation of hypothalamic mTOR signaling in the hypothalamus through its phosphorylation.

### 1.2. Results of the oral administration of D-Chiroinositol (500 mg/kg) dissolved in sterile water to adult male Wistar Rats

As shown in figure 4, oral administration of D-Chiroinositol (500 mg/kg) enhances Ghrelin secretion as measured through monitorization of circulating plasma Ghrelin concentrations.

### 1.3. Proposed model of action of inositols in metabolic aging

Either D-Pinitol or D-Chiroinositol enhance Ghrelin secretion and promote a metabolic situation characterized by reduced insulin demand from endocrine pancreas, neoglucogenesis in the liver, enhanced muscle use of glucose with associated muscle growth, and enhanced mTOR signaling in the hypothalamus leading to appetite increase. The overall consequences of this unique pharmacological profile might include pancreas protection from exhaustion derived of age-associated insulin resistance and obesity, enhanced muscular vitality (preventing the characteristic sarcopenia and frailty of the elderly) and a better directioning of glucose disposal by the body.

### References

1.- Bettedi L, Foukas LC. Growth factor, energy and nutrient sensing signaling pathways in metabolic ageing. Biogerontology. 2017 Dec;18(6):913-929.
2.- Soriguer F, et al. Prevalence of diabetes mellitus and impaired glucose regulation in Spain: the Di@bet.es Study. Diabetologia. 2012 Jan;55(1):88-93.
3.- Kojima M, Hosoda H, Date Y, Nakazato M, Matsuo H, Kangawa K (December 1999). "Ghrelin is a growth-hormone-releasing acylated peptide from stomach". Nature. 402 (6762): 656-60
4.- Seim I, Amorim L, Walpole C, Carter S, Chopin LK, Herington AC (January 2010). "Ghrelin gene-related peptides: multifunctional endocrine / autocrine modulators in health and disease". Clinical and Experimental Pharmacology & Physiology. 37 (1): 125-31
5.- Strasser F. Clinical application of ghrelin. Curr Pharm Des. 2012;18(31):4800-12.
6.- Castañeda TR, Tong J, Datta R, Culler M, Tschöp MH (January 2010). "Ghrelin in the regulation of body weight and metabolism". Frontiers in Neuroendocrinology. 31 (1): 44-60.
7.- Inui A, Asakawa A, Bowers CY, Mantovani G, Laviano A, Meguid MM, Fujimiya M (March 2004). "Ghrelin, appetite, and gastric motility: the emerging role of the stomach as an endocrine organ". The FASEB Journal. 18 (3): 439-56.
8.- Alamri BN, Shin K, Chappe V, Anini Y. The role of ghrelin in the regulation of glucose homeostasis. Horm Mol Biol Clin Investig. 2016 Apr 1;26(1):3-11.
9.- Pena-Bello L, Pertega-Diaz S, Outeiriño-Blanco E, Garcia-Buela J, Tovar S, Sangiao-Alvarellos S, Dieguez C, Cordido F. Effect of oral glucose administration on rebound growth hormone release in normal and obese women: the role of adiposity, insulin sensitivity and ghrelin. PLoS One. 2015 Mar 17;10(3):e0121087.
10.- Leinonen T, Antero Kesäniemi Y, Hedberg P, Ukkola O. Serum ghrelin and prediction of metabolic parameters in over 20-year follow-up. Peptides. 2016 Feb;76:51-6.
11.- Martins L, Fernandez-Mallo D, Novelle MG, Vázquez MJ, Tena-Sempere M,Nogueiras R, López M, Diéguez C. Hypothalamic mTOR signaling mediates the orexigenic action of ghrelin. PLoS One. 2012;7(10):e46923.
12.- Yada T, Damdindorj B, Rita RS, Kurashina T, Ando A, Taguchi M, Koizumi M, Sone H, Nakata M, Kakei M, Dezaki K. Ghrelin signalling in β-cells regulates insulin secretion and blood glucose. Diabetes Obes Metab. 2014 Sep;16 Suppl 1:111-7.
13.- Kurashina T, Dezaki K, Yoshida M, Sukma Rita R, Ito K, Taguchi M, Miura R, Tominaga M, Ishibashi S, Kakei M, Yada T. The β-cell GHSR and downstream cAMP/TRPM2 signaling account for insulinostatic and glycemic effects of ghrelin. Sci Rep. 2015 Sep 15;5:14041.
14.- Dang NT, Mukai R, Yoshida K, Ashida H. D-pinitol and myo-inositol stimulate translocation of glucose transporter 4 in skeletal muscle of C57BL/6 mice. Biosci Biotechnol Biochem. 2010;74(5):1062-7.
15.- Yap A, Nishiumi S, Yoshida K, Ashida H. Rat L6 myotubes as an in vitro model system to study GLUT4-dependent glucose uptake stimulated by inositol derivatives. Cytotechnology. 2007 Dec;55(2-3):103-8.
16.- Nass R, Farhy LS, Liu J, Pezzoli SS, Johnson ML, Gaylinn BD, Thorner MO. Age-dependent decline in acyl-ghrelin concentrations and reduced association of acyl-ghrelin and growth hormone in healthy older adults. J Clin Endocrinol Metab. 2014 Feb;99(2):602-8.

## Claims

1. A composition, a pharmaceutical composition or a nutraceutical or food composition, comprising D-pinitol, D-Chiro inositol or myo-inositol or any salt thereof, for use in the treatment or prevention of disorders, diseases or conditions **characterized by** an impaired ghrelin secretion resulting in active ghrelin blood circulation levels lower than normal pre-prandial active ghrelin blood circulation reference levels in a subject in need thereof.

2. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are age related conditions, and wherein said age related conditions are selected from the list consisting of loss of muscle mass and increased muscle frailty, mild cognitive impairment, atherosclerosis, cardiovascular disease, type 2 diabetes, osteoporosis, hypertension, Alzheimer's disease, arthritis, cataracts and cancer.

3. A non-therapeutic use of a composition, preferably a nutraceutical or food composition, comprising D-pinitol, D-Chiro inositol or myo-inositol or any acceptable salt thereof, for promoting appetite.

4. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are age related conditions, and wherein said age related conditions consists of loss of muscle mass and increased muscle frailty.

5. A non-therapeutic use of a composition, preferably a nutraceutical or food composition, comprising D-pinitol, D-Chiro inositol or myo-inositol or any acceptable salt thereof, for increasing net muscle mass.

6. The composition for use according to claim 1, wherein said disorders, diseases or conditions **characterized by** an impaired ghrelin secretion resulting in active ghrelin blood circulation levels lower than normal pre-prandial active ghrelin blood circulation reference levels is sarcopenia.

7. The composition for use according to claim 1, wherein said disorders, diseases or conditions **characterized by** an impaired ghrelin secretion resulting in active ghrelin blood circulation levels lower than normal pre-prandial active ghrelin blood circulation reference levels is hypertension, preferably age related hypertension.

8. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are age related conditions, and wherein said age related conditions consists of diabetes type 2, hypertension, dyslipidaemia, cardiovascular disease, non-alcoholic steatohepathitis and brain insulin resistance associated to Alzheimer 's disease.

9. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are age related conditions, wherein said age related conditions consists of the physiological decline in growth hormone release associated with aging.

10. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are age related conditions, wherein said age related conditions consists of cognitive decline.

11. A non-therapeutic use of a composition, preferably a nutraceutical or food composition, comprising D-pinitol, D-Chiro inositol or myo-inositol or any acceptable salt thereof, for improving cognition.

12. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are selected from the list consisting of Azlheimer's disease, vascular dementia, Parkinson's Disease, and Huntington's disease.

13. The composition for use according to claim 1, wherein said disorders, diseases or conditions **characterized by** an impaired ghrelin secretion resulting in active ghrelin blood circulation levels lower than normal pre-prandial active ghrelin blood circulation reference levels are selected from the list consisting of diabetes mellitus, preferably type II diabetes.

14. The composition for use according to any of claims 1, 2, 4, 6, 7, 8 to 10, 12 and 13, wherein said composition is a pharmaceutical composition which further comprises a pharmaceutically acceptable carrier.

15. The composition for use according to any of claims 1 to 14, wherein said composition is administered orally or via intragastric.
